# EUROPÄISCHE PATENTANMELDUNG

(11) **EP 0 561 357 A1**
(43) Veröffentlichungstag der Anmeldung: **22.09.1993**
(21) Anmeldenummer: 93104284.0
(22) Anmeldetag: 16.03.1993
(51) Int. Cl.: A61K 45/06, A61K 31/44, A61K 31/50

(54) **Pharmazeutische Zubereitung enthaltend einen Kalium Kanalaktivator und einen Angiotensin II-Rezeptorantagonisten**

(30) Priorität: 20.03.1992 DE 4209071
(71) Anmelder: MERCK PATENT GmbH, D-64271 Darmstadt (DE)
(72) Erfinder: Mederski, Werner, Dr., W-6106 Erzhausen (DE); Dorsch, Dieter, Dr., W-6105 Ober-Ramstadt (DE); Gericke, Rolf, Dr., W-6104 Seeheim (DE); Baumgarth, Manfred, Dr., W-6100 Darmstadt (DE); Schelling, Pierre, Prof. Dr., W-5109 Mühltal (DE); Bergmann, Rolf, Dr., 64385 Reichelsheim (DE); Beier, Norbert, Dr., W-6107 Reinheim 5 (DE); Lues, Ingeborg, Dr., W-6100 Darmstadt (DE); Minck, Klaus-Otto, Dr., W-6105 Ober-Ramstadt (DE)

(57) **Zusammenfassung**

Die Erfindung betrifft eine pharmazeutische Zubereitung enthaltend einen Kaliumkanalaktivator und einen Angiotensin II-Rezeptorantagonisten.

## Beschreibung

Gegenstand der Erfindung ist eine neue pharmazeutische Zubereitung enthaltend einen Kaliumkanalaktivator ("KKA") und einen Angiotensin II-Rezeptorantagonisten ("AR").

Eine besonders bevorzugte Ausführungsform ist dadurch gekennzeichnet, daß sie einen KKA der Formel I enthält: 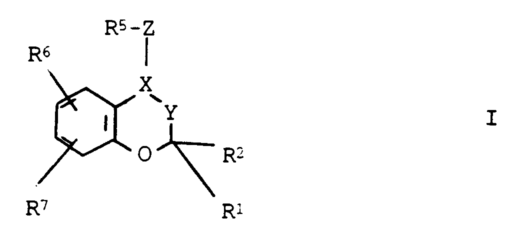
worin
- X-Y: 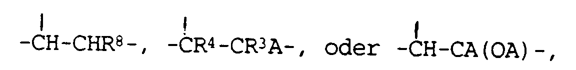 sowie, falls der Rest R⁵ weder vollständig noch partiell hydriert ist, auch 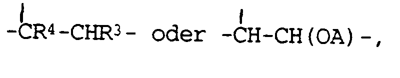
- R¹: A,
- R² und R⁸: jeweils H oder A,
- R¹ und R²: zusammen auch Alkylen mit 3-6 C-Atomen,
- R³: OH oder OAc,
- R⁴: H,
- R³ und R⁴: zusammen auch eine Bindung,
- R⁵: einen unsubstituierten oder einen ein- oder zweifach durch A, F, Cl, Br, J, OH, OA, OAc, NO₂, NH₂, AcNH, HOOC und/oder AOOC substituierten Pyridyl-, Pyridazinyl-, Pyrimidinyl-, Pyrazinyl-, Oxo-dihydro-pyridyl-, Oxo-dihydro-pyridazinyl-, Oxo-dihydro-pyrimidinyl-, Oxo-dihydro-pyrazinyl-, 3H- oder 5H-2-Pyrrolinon-1-yl-, 2H-1-Isochinolinon-2-yl-, 2H-1-Phthalazinon-2-yl-, 3H-4-Chinazolinon-3-yl- oder 1H-2-Thiopyridon-1-yl-rest, wobei diese Reste auch vollständig oder partiell hydriert sein können,
- R⁶ und R⁷: jeweils H, A, HO, AO, CHO, ACO, ACS, HOOC, AOOC, AO-CS, ACOO, A-CS-O, Hydroxy-alk, Mercapto-alk, NO₂, NH₂, NHA, NA₂, CN, F, Cl, Br, J, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alk, Nitro-alk, Cyan-alk, A-C(=NOH) oder A-C(=NH₂),
- Z: eine Bindung, O, S oder NH,
- R⁷: auch Pyridyl, Pyridazinyl, Pyrimidinyl oder Pyrazinyl,
- A: Alkyl mit 1-6 C-Atomen (wobei mehrere Gruppen A unabhängig voneinander verschiedene Alkylgruppen bedeuten können),
- -alk-: Alkylen mit 1-6 C-Atomen und
- Ac: Alkanoyl mit 1-8 C-Atomen oder Aroyl mit 7-11 C-Atomen
bedeuten,
und/oder eines seiner physiologischen unbedenklichen Salze.

Die Verbindungen der Formel I sind teilweise bekannt (vgl. z.B. DE-A-37 26 261).

Unter den Verbindungen der Formel I sind diejenigen bevorzugt, in denen die Reste R¹ und R² jeweils CH₃, der Rest R⁷ H und/oder der Rest R⁶ CN bedeuten, wobei der Rest R⁶ vorzugsweise in 6-Stellung steht. Die Gruppierung X-Y bedeutet vorzugsweise 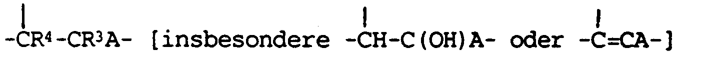
oder, falls der Rest R⁵ weder vollständig noch partiell hydriert ist, auch

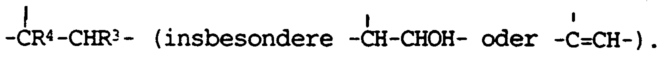

Die Gruppe R⁵-Z bedeutet vorzugsweise 1H-2-Pyridon-1-yl, 2-Hydroxy-4-pyridyl-oxy, 6-Hydroxy-3-pyridazinyl-oxy, 1,6-Dihydro-1-methyl- oder 1,6-Dihydro-1-ethyl-6-oxo-3-pyridazinyl-oxy.

Einzelne bevorzugte Verbindungen der Formel I sind 2,2-Dimethyl-4-(1H-2-pyridon-1-yl)-6-cyan-3-chromanol (Ia), insbesondere dessen (-)-Enantiomeres, 2,2-Dimethyl-4-(1H-2-pyridon-1-yl)-6-cyan-2H-chromen ("Bimakalim"; Ib), 2,2-Dimethyl-4-(6-hydroxy-3-pyridazinyl-oxy)-6-cyan-3-chromanol, insbesondere dessen (-)-Enantiomeres, 2,2,3-Trimethyl-4-(6-hydroxy-3-pyridazinyl-oxy)-6-cyan-3-chromanol, insbesondere dessen (-)-Enantiomeres, 2,2-Dimethyl-4-(1,6-dihydro-1-methyl-6-oxo-3-pyridazinyl-oxy)-6-cyan-3-chromanol (Ic), insbesondere dessen (-)-Enantiomeres.

AR, insbesondere Angiotensin II-Subtyp 1-Rezeptorantagonisten ("AT₁-Antagonisten"), sind Substanzen, die das Hormon Angiotensin II von seinem Rezeptor (AT₁-Rezeptor) verdrängen. AR, insbesondere AT₁-Antagonisten senken den Blutdruck, wenn die Hypertonie mit einer erhöhten Aktivität des Renin-Angiotensin-Systems verbunden ist.

Bevorzugte AR sowie Verfahren zu ihrer Herstellung sind z.B. in folgenden Patentanmeldungen angegeben. DE-40 06 693, EP-28 833, EP-230 922, EP-245 637, EP-253 310, EP-323 841, EP-324 377, EP-392 317, EP-399 731, EP-400 835, EP-400 974, EP-401 030, EP-403 158, EP-403 159, EP-407 102, EP-407 342, EP-409 332, EP-411 507, EP-411 766, EP-412 594, EP-415 886, EP-419 048, EP-420 237, EP-424 317, EP-425 211, EP-425 921, EP-426 021, EP-427 463, EP-429 257, EP-430 300, EP-430 709, EP-432 737, EP-434 038, EP-434 249, EP-435 827, EP-437 103, EP-438 869, EP-442 473, EP-443 568, EP-443 983, EP-445 811, EP-446 062, EP-449 699, EP-450 566, EP-453 210, EP-454 831, EP-456 442, EP-456 510, EP-459 136, EP-461 039, EP-461 040, US-4 880 804, US-4 916 129, US-5 041 552, US-5 045 540, US-5 049 565, US-5 053 329, WO-83 03 250, WO-87 05 029, WO-91 00 277, WO-91 00 281, WO-91 07 404, WO-91 09 847, WO-91 10 140, WO-91 11 909, WO-91 11 999, WO-91 12 001, WO-91 12 002, WO-91 12 271, WO-91 13 088, WO-91 14 367, WO-91 14 679, WO-91 15 206, WO-91 15 209, WO-91 15 479, WO-91 16 313, WO-91 17 148.

Weitere bevorzugte AR entsprechen der Formel II 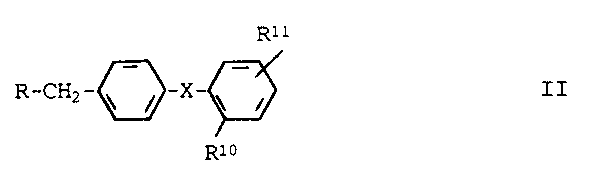
worin 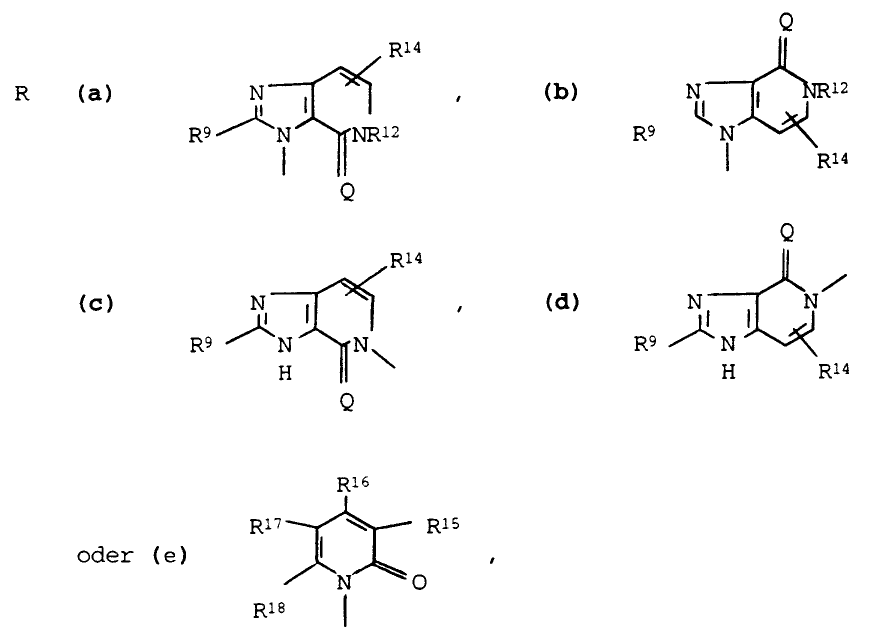
- R⁹: A, Alkenyl oder Alkinyl mit jeweils bis zu 6 C-Atomen,
- R¹⁰: H, COOH, COOA, CN, NO₂, NH₂, NHCOR¹³, NHSO₂R¹³ oder 1H-5-Tetrazolyl,
- R¹¹: H, Hal, A, OA oder NO₂,
- R¹²: H, R¹³, Cyan-alk-, AOOC-alk-, Carboxy-alk-, 1H-5-Tetrazolyl-alk-, Ar-alk-, R¹³-CO-alk-, Ar-CO-alk-, Het-CO-alk-, Het-alk-, Alkenyl oder Alkinyl mit jeweils bis zu 6 C-Atomen, 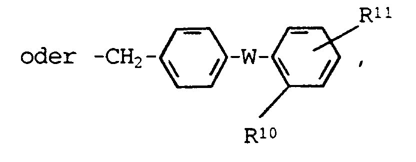
- R¹³: Alkyl mit 1-4 C-Atomen, worin auch ein oder mehrere H-Atom(e) durch F ersetzt sein können,
- R¹⁴: H oder Hal,
- R¹⁵: H, COOH, COOA, CHO, CN, NO₂, CH₂R¹⁹, CH₂OR²⁰, NR²¹R²², CH₂NR²¹R²², CONR²¹R²² oder 1H-5-Tetrazolyl,
- R¹⁶: H oder A,
- R¹⁷, R¹⁸ und R²¹: jeweils H, A, Alkenyl mit 2-6 C-Atomen, Alkinyl mit 2-6 C-Atomen, Ar oder Ar-alk-,
- R¹⁹: H, Hal, A, Ar, CN, COOH, COOA, CH₂COOH, CH₂COOA oder 1H-5-Tetrazolyl,
- R²⁰: H, A, Ar, Ar-alk-, COA, COAr, COOA, COOAr, CONR²³R²⁴, COO-alk-Ar oder A-O-alk-,
- R²¹: H, A, ein- oder mehrfach durch F substituiertes A, Ar, Ar-alk-, CO-A, CO-Ar, CO-alk-Ar, COOA, COOAr, COO-alk-Ar oder CONR²³R²⁴,
- NR²¹R²²: auch Pyrrolidino, Piperidino, Morpholino, Succinimido oder Phthalimido,
- R²³ und R²⁴: jeweils H, A, Cycloalkyl mit 3-8 C-Atomen, Alkenyl mit 2-6 C-Atomen, Alkinyl mit 2-6 C-Atomen oder Ar,
- W: fehlt, -CO-, -O-, -NH-CO-, -CO-NH-, -CH₂-O-, -O-CH₂-, -O-CH(COOH)-, -NH-CH(COOH)-, -NA-CH(COOH)-, -CH=C(COOH)-, -CH=C(CN)- oder -CH=C(1H-5-tetrazolyl)-,
- Q: O oder S,
- Ar: eine unsubstituierte oder eine ein- oder zweifach durch Hal, R¹³, OH, OR¹³, COOH, COOR¹³, CN, NO₂, NH₂, NHA, N(A)₂, NHCOR¹³, NHCOOA, NHSO₂R¹³ und/oder 1H-5-Tetrazolyl substituierte Phenylgruppe,
- Het: einen 5- oder 6-gliedrigen heteroaromatischen Rest mit 1 bis 3 H-, O- und/oder S-Atomen, der auch mit einem Benzol- oder Pyridinring kondensiert sein kann,
- Hal: F, Cl, Br oder I bedeuten und
A und -alk- die bei Formel I angegebenen Bedeutungen haben,
sowie ihren Salzen.

Weiterhin bevorzugte AR entsprechen der Formel III 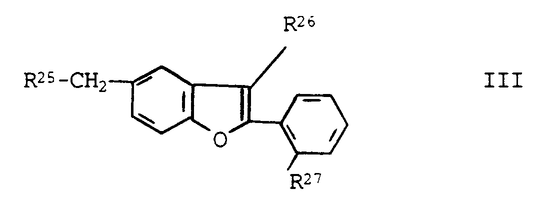
worin
- R²⁵: den Rest 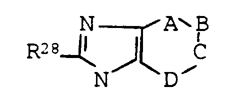
- R²⁶: H, Hal, COOH, CONH₂, CHO, CH, NH₂ oder 1H-5-Tetrazolyl,
- R²⁷: H, COOH, COOR²⁸, CH, NO₂, NH₂, NHCOCF₃, NHSO₂CF₃ oder 1H-5-Tetrazolyl,
- R²⁸: H, A, Alkenyl oder Alkinyl mit jeweils bis zu 6 C-Atomen,
- -A-B-C-D-: eine der Gruppen -CH=CH-CH=N-, -CH=CH-N=CH-, -CH=N-CH=CH-, -N=CH-CH=CH-, -CH=CH-CO-NR²⁹-, -CH=CH-NR²⁹-CO-, -CO-NR²⁹-CH=CH- oder -NR²⁹-CO-CH=CH-, worin die H-Atome der -CH-Gruppen durch A, Hal, COOR²⁸, CN und/oder 1H-5-Tetrazolyl substituiert sein können,
- R²⁹: H, A, Cyan-alk-, R²⁸OOC-alk-, 1H-5-Tetrazolyl-alk- oder Ar-alk- bedeuten und
A, -alk-, Ar und Hal die bei Formel I oder II angegebenen Bedeutungen haben, sowie ihren Salzen.

Einige bevorzugte AR im einzelnen sind:
2-Butyl-4-chlor-5-hydroxymethyl-1-[2'-(1H-5-tetrazolyl)-biphenylyl-4-methyl]-imidazol und dessen K-Salz ("DUP 753"); 6-Butyl-1,2-dihydro-2-oxo-1-[2'-(1H-5-tetrazolyl)-biphenylyl-4-methyl]-pyridin (F. 138°);
sowie die folgenden 2-Butyl-4,5-dihydro-4-oxo-3-[2'-(1H-5-tetrazolyl)-biphenylyl-4-methyl]-3H-imidazo[4,5-c]pyridine:
5-o-Fluorbenzyl- (F. 118°)
5-(2-Thienylmethyl)- (F. 145°)
5-(3,3-Dimethyl-2-oxo-butyl)- (F. 203°)
5-(o-Methoxyphenacyl)- (F. 137°)
5-(o-Methoxycarbonylbenzyl)- (F. 124°).

Gleichzeitige Gabe von AR verstärkt bemerkenswerterweise deutlich die blutdrucksenkende Wirkung der genannten KKA, senkt die Herzfrequenz und vermindert die Aktivität des Renin-Angiotensin-Systems. Dieser Effekt kann z.B. in Standardtests an narkotisierten oder wachen Ratten, Hunden, Katzen, Affen oder Minischweinen ermittelt werden, z.B. nach Methoden, wie sie in der EP-A2-0 271 271 beschrieben sind.

Die neue pharmazeutische Zubereitung kann hergestellt werden, indem man (mindestens) einen KKA und (mindestens) einen AR zusammen mit mindestens einem festen, flüssigen oder halbflüssigen Träger- oder Hilfsstoff in eine geeignete Dosierungsform bringt. Die so erhaltenen Zubereitungen können als Arzneimittel in der Human- oder Veterinärmedizin, insbesondere bei der Senkung des Blutdrucks, eingesetzt werden. Als Trägersubstanzen kommen organische oder anorganische Stoffe in Frage, die sich für die enterale (z.B. orale oder rektale), parenterale oder topische Applikation eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen, zur rektalen Anwendung Suppositorien, zur parenteralen Applikation Lösungen, vorzugsweise ölige oder wässerige Lösungen, ferner Suspensionen, Emulsionen oder Implantate, für die topische Anwendung Salben, Cremes oder Pflaster. Die Wirkstoffe können auch lyophilisiert und die erhaltenen Lyophilisate z.B. zur Herstellung von Injektionspräparaten verwendet werden. Die Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können auch weitere Wirkstoffe enthalten, z.B. andere blutdrucksenkende oder diuretisch wirkende Stoffe.

Die erfindungsgemäßen Zubereitungen werden in der Regel zur Therapie und/oder Prophylaxe von Störungen des kardiovaskulären Systems, insbesondere dekompensierter Herzinsuffizienz, Angina pectoris, peripheren oder zerebralen Gefäßerkrankungen sowie Krankheitszuständen, die mit Bluthochdruck verbunden sind, in Analogie zu bekannten blutdrucksenkend wirksamen Substanzen, insbesondere den AR selbst, verabreicht. Die Dosierungen der KKA liegen vorzugsweise zwischen etwa 0,01 mg und 50 mg, insbesondere 0,02 und 5 mg, ganz besonders bevorzugt zwischen 0,1 und 1 mg pro Dosierungseinheit. Die AR werden vorzugsweise in Dosierungen zwischen 0,5 und 500 mg, insbesondere zwischen 1 und 200 mg pro Dosierungseinheit verwendet. Bevorzugte Dosierungen der angegebenen einzelnen AR liegen zwischen 2 und 200, insbesondere zwischen 5 und 100 mg pro Dosierungseinheit. Die tägliche Dosierung der KKA liegt vorzugsweise zwischen etwa 0,0001 und 1, vorzugsweise zwischen 0,0002 und 0,1 mg/kg Körpergewicht, diejenige der AR vorzugsweise zwischen etwa 0,01 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Bestandteile der neuen pharmazeutischen Zubereitung werden vorzugsweise kombiniert verabreicht. Sie können aber auch einzeln gleichzeitig oder aufeinanderfolgend verabreicht werden.

### Beispiel A: Tabletten

Ein Gemisch von 20 g KKA-Wirkstoff (z.B. Ia) 0,4 kg AR-Wirkstoff (z.B. DUP 753), 4 kg Lactose, 1,2 kg Kartoffelstärke, 0,2 kg Talk und 0,1 kg Magnesiumstearat wird in üblicher Weise zu Tabletten gepreßt, derart, daß jede Tablette 1 mg KKA-Wirkstoff und 20 mg AR-Wirkstoff enthält.

### Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C: Kapseln

Man füllt ein gesiebtes Gemisch von 30 g KKA-Wirkstoff (z.B. Ib), 1 kg AR-Wirkstoff und 6 kg Lactose in üblicher Weise in Hartgelatinekapseln, so daß jede Kapsel 0,3 mg KKA-Wirkstoff und 10 mg AR-Wirkstoff enthält.

### Beispiel D: Ampullen

Eine Lösung von 2 g KKA-Wirkstoff (z.B. Ic) und 0,1 kg AR-Wirkstoff in 30 l zweifach destilliertem Wasser wird steril filtriert, in Ampullen abgefüllt, unter sterilen Bedingungen lyophilisiert und steril verschlossen. Jede Ampulle enthalt 0,1 mg KKA-Wirkstoff und 5 mg AR-Wirkstoff.

## Patentansprüche

1. Pharmazeutische Zubereitung enthaltend einen Kaliumkanalaktivator und einen Angiotensin II-Rezeptorantagonisten.

2. Pharmazeutische Zubereitung nach Patentanspruch 1, dadurch gekennzeichnet, daß sie einen Kaliumkanalaktivator der Formel I enthält: 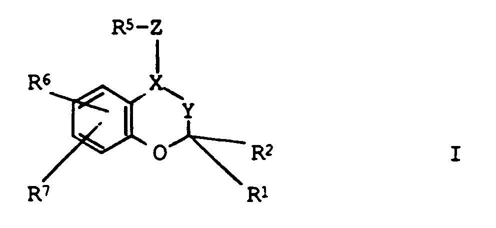 worin
X-Y 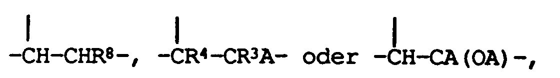 sowie, falls der Rest R⁵ weder vollständig noch partiell hydriert ist, auch 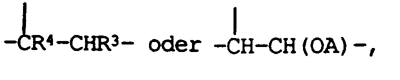
R¹ A,
R² und R⁸ jeweils H oder A,
R¹ und R² zusammen auch Alkylen mit 3-6 C-Atomen,
R³ OH oder OAc,
R⁴ H.
R³ und R⁴ zusammen auch eine Bindung,
R⁵ einen unsubstituierten oder einen ein- oder zweifach durch A, F, Cl, Br, J, OH, OA, OAc, NO₂, NH₂, AcNH, HOOC und/oder AOOC substituierten Pyridyl-, Pyridazinyl-, Pyrimidinyl-, Pyrazinyl-, Oxo-dihydropyridyl-, Oxo-dihydro-pyradazinyl, Oxo-dihydro-pyrimidinyl-, Oxo-dihydro-pyrazinyl, 3H- oder 5H-2-Pyrrolinon-1-yl-, 2H-1-Isochinolinon-2-yl-, 2H-2-Phthalazinon-2-yl, 3H-4-Chinazolinon-3-yl- oder 1H-2-Thiopyridon-1-yl-rest, wobei diese Rest auch vollständig oder partiell hydriert sein können,
R⁶ und R⁷ jeweils H, A, HO, AO, CHO, ACO, ACS, HOOC, AOOC, AO-CS, ACOO, A-CS-O, Hydroxy-alk-, Mercapto-alk-, NO₂, NH₂, NHA, NA₂, CN, F, Cl, Br, J, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alk-, Nitro-alk-, Cyan-alk-, A-C(=NOH) oder A-C(=NNH₂),
Z eine Bindung, O, S oder NH,
R⁷ auch Pyridyl, Pyridazinyl, Pyrimidinyl oder Pyrazinyl,
A Alkyl mit 1-6 C-Atomen (wobei mehrere Gruppen A unabhängig voneinander verschiedene Alkylgruppen bedeuten können),
-alk- Alkylen mit 1-6 C-Atomen und
Ac Alkanoyl mit 1-8 C-Atomen oder Aroyl mit 7-11 C-Atomen
bedeuten,
und/oder eines seiner physiologisch unbedenklichen Salze.

3. Verfahren zur Herstellung einer pharmazeutischen Zubereitung nach Anspruch 1, dadurch gekennzeichnet, daß man einen Kaliumkanalaktivator und einen Angiotensin II-Rezeptorantagonisten zusammen mit mindestens einem festen, flüssigen oder halbflüssigen Träger- oder Hilfsstoff in eine geeignete Dosierungsform bringt.

4. Verwendung einer pharmazeutischen Zubereitung nach Anspruch 1 bei der Senkung des Blutdrucks.
